# EUROPEAN PATENT SPECIFICATION

(11) **EP 0 726 313 B1**
(45) Date of publication and mention of the grant of the patent: **14.01.2004**
(21) Application number: 96300803.2
(22) Date of filing: 07.02.1996
(51) Int. Cl.: C12N 15/12, C12N 15/55

(54) **DNA coding for mammalian L-asparaginase**
DNS, die für L-Asparaginase von Säugetieren kodiert
ADN codant pour la L-asparaginase de mammifères

(30) Priority: 08.02.1995 JP 4256495
(43) Date of publication of application: 14.08.1996
(73) Proprietor: KABUSHIKI KAISHA HAYASHIBARA SEIBUTSU KAGAKU KENKYUJO, Okayama-shi Okayama (JP)
(72) Inventor: Ario, Takeshi, Okayama-shi, Okayama (JP); Taniai, Madoka, Okayama-shi, Okayama (JP); Torigoe, Kakuji, Kurashiki-shi, Okayama (JP); Kurimoto, Masashi, Okayama-shi, Okayama (JP)
(74) Representative: Daniels, Jeffrey Nicholas

(56) References cited:
- GENE, vol. 78, 1989, pages 37-46, XP002012972 P.G. JERLSTROM ET AL.: "Structure and expression in E. coli K-12 of the L-asparaginase I-encoding ansA gene and its flanking regions"
- GENE, vol. 91, 1990, pages 101-105, XP002012973 D.T: BONTHRON: "L-asparaginase II of E. coli K-12: cloning, mapping and sequencing of the ansB gene"
- BIOCHEMISTRY, vol. 5, no. 5, 1966, pages 1605-1612, XP002012974 T.O. YELLIN ET AL.: "Purification and proeperties of guinea pig serum asparaginase."
- Suld and Herbut 1970 JBC 245:2797-2801 XP000600899
- Stewart et al. 1995 JBC 270:25-28 XP000577063
- REECK ET AL: 'HOMOLOGY IN PROTEINS AND NUCLEIC ACIDS: A TERMINOLOGY MUDDLE AND A WAY OUT OF IT' CELL vol. 50, 1987, page 667, XP002913075
- RYOYAMA C.: 'EFFECT OF STRAIN DIFFERENCES ON HEAT-SUSCEPTIBILITY OF L-ASPARAGINASE IN THE GUINEA PIG' JAPANESE JOURNAL OF EXPERIMENTAL MEDICINE vol. 48, no. 4, 01 August 1978, pages 327 - 336, XP000600736

### SEQUENCE LISTING

(1) GENERAL INFORMATION:
   (i) APPLICANT:
      NAME:KABUSHIKI KAISHA HAYASHIBARA SEIBUTSU KAGAKU KENKYUJO
   (ii) TITLE OF INVENTION:DNA CODING FOR MAMMALIAN L-ASPARAGINASE
   (iii) NUMBER OF SEQUENCES:11
   (iv) ADDRESS:
      (A) ADDRESSEE:KABUSHIKI KAISHA HAYASHIBARA SEIBUTSU KAGAKU KENKYUJO
      (B) STREET:2-3, 1-CHOME, SHIMOISHII
      (C) CITY:OKAYAMA
      (E) COUNTRY:JAPAN
      (F) POSTAL CODE (ZIP) :700
   (v) COMPUTER READABLE FORM:
      (A) MEDIUM TYPE:Floppy disk
      (B) COMPUTER:IBM PC compatible
      (C) OPERATING SYSTEM:PC-DOS/MS-DOS
   (vii) PRIOR APPLICATION DATA:
      (A1) APPLICATION NUMBER:JP 42564/95 ,
      (B1) FILING DATE:February 8, 1995
(2) INFORMATION FOR SEQ ID NO:1:
   (i) SEQUENCE CHARACTERISTICS:
      (A) LENGTH:4 amino acids
      (B) TYPE:amino acid
      (D) TOPOLOGY:linear
   (ii) MOLECULE TYPE:peptide
   (xi) SEQUENCE DESCRIPTION:SEQ ID NO:1:
   Thr Gly Gly Thr
   1
(3) INFORMATION FOR SEQ ID NO:2:
   (i) SEQUENCE CHARACTERISTICS:
      (A) LENGTH:5 amino acids
      (B) TYPE:amino acid
      (D) TOPOLOGY:linear
   (ii) MOLECULE TYPE:peptide
   (xi) SEQUENCE DESCRIPTION:SEQ ID NO:2:
   His Gly Thr Asp Thr
   1               5
(4) INFORMATION FOR SEQ ID NO:3:
   (i) SEQUENCE CHARACTERISTICS:
      (A) LENGTH:565 amino acids
      (B) TYPE:amino acid
      (D) TOPOLOGY:linear
   (ii) MOLECULE TYPE:peptide
      (xi) SEQUENCE DESCRIPTION:SEQ ID NO:3:
   Met Ala Arg Ala Ser Gly Ser Glu Arg His Leu Leu Leu Ile Tyr Thr Gly
   1 5 10 15
   Gly Thr Leu Gly Met Gln Ser Lys Gly Gly Val Leu Val Pro Gly Pro Gly
   20 25 30
   Leu Val Thr Leu Leu Arg Thr Leu Pro Met Phe His Asp Lys Glu Phe Ala
   35 40 45 50
   Gln Ala Gln Gly Leu Pro Asp His Ala Leu Ala Leu Pro Pro Ala Ser His
   55 60 65
   Gly Pro Arg Val Leu Tyr Thr Val Leu Glu Cys Gln Pro Leu Leu Asp Ser
   70 75 80 85
   Ser Asp Met Thr Ile Asp Asp Trp Ile Arg Ile Ala Lys Ile Ile Glu Arg
   90 95 100
   His Tyr Glu Gln Tyr Gln Gly Phe Val Val Ile His Gly Thr Asp Thr Met
   105 110 115
   Ala Phe Gly Ala Ser Met Leu Ser Phe Met Leu Glu Asn Leu His Lys Pro
   120 125 130 135
   Val Ile Leu Thr Gly Ala Gln Val Pro Ile Arg Val Leu Trp Asn Asp Ala
   140 145 150
   Arg Glu Asn Leu Leu Gly Ala Leu Leu Val Ala Gly Gln Tyr Ile Ile Pro
   155 160 165 170
   Glu Val Cys Leu Phe Met Asn Ser Gln Leu Phe Arg Gly Asn Arg Val Thr
   175 180 185
   Lys Val Asp Ser Gln Lys Phe Glu Ala Phe Cys Ser Pro Asn Leu Ser Pro
   190 195 200
   Leu Ala Thr Val Gly Ala Asp Val Thr Ile Ala Trp Asp Leu Val Arg Lys
   205 210 215 220
   Val Asn Trp Lys Asp Pro Leu Val Val His Ser Asn Met Glu His Asp Val
   225 230 235
   Ala Leu Leu Arg Leu Tyr Pro Gly Ile Pro Ala Ser Leu Val Arg Ala Phe
   240 245 250 255
   Leu Gln Pro Pro Leu Lys Gly Val Val Leu Glu Thr Phe Gly Ser Gly Asn
   260 265 270
   Gly Pro Ser Lys Pro Asp Leu Leu Gln Glu Leu Arg Ala Ala Ala Gln Arg
   275 280 285
   Gly Leu Ile Met Val Asn Cys Ser Gln Cys Leu Arg Gly Ser Val Thr Pro
   290 295 300 305
   Gly Tyr Ala Thr Ser Leu Ala Gly Ala Asn Ile Val Ser Gly Leu Asp Met
   310 315 320
   Thr Ser Glu Ala Ala Leu Ala Lys Leu Ser Tyr Val Leu Gly Leu Pro Glu
   325 330 335 340
   Leu Ser Leu Glu Arg Arg Gln Glu Leu Leu Ala Lys Asp Leu Arg Gly Glu
   345 350 355
   Met Thr Leu Pro Thr Ala Asp Leu His Gln Ser Ser Pro Pro Gly Ser Thr
   360 365 370
   Leu Gly Gln Gly Val Ala Arg Leu Phe Ser Leu Phe Gly Cys Gln Glu Glu
   375 380 385 390
   Asp Ser Val Gln Asp Ala Val Met Pro Ser Leu Ala Leu Ala Leu Ala His
   395 400 405
   Ala Gly Glu Leu Glu Ala Leu Gln Ala Leu Met Glu Leu Gly Ser Asp Leu
   410 415 420 425
   Arg Leu Lys Asp Ser Asn Gly Gln Thr Leu Leu His Val Ala Ala Arg Asn
   430 435 440
   Gly Arg Asp Gly Val Val Thr Met Leu Leu His Arg Gly Met Asp Val Asn
   445 450 455
   Ala Arg Asp Arg Asp Gly Leu Ser Pro Leu Leu Leu Ala Val Gln Gly Arg
   460 465 470 475
   His Arg Glu Cys Ile Arg Leu Leu Arg Lys Ala Gly Ala Cys Leu Ser Pro
               480                 485                 490
   Gln Asp Leu Lys Asp Ala Gly Thr Glu Leu Cys Arg Leu Ala Ser Arg Ala
       495                 500                 505                 510
   Asp Met Glu Gly Leu Gln Ala Trp Gly Gln Ala Gly Ala Asp Leu Gln Gln
                   515                 520                 525
   Pro Gly Tyr Asp Gly Arg Ser Ala Leu Cys Val Ala Glu Ala Ala Gly Asn
           530                 535                 540
   Gln Glu Val Leu Ala Leu Leu Arg Asn Leu Ala Leu Val Gly Pro Glu Val
   545                 550                 555                 560
   Pro Pro Ala Ile
               565
(5) INFORMATION FOR SEQ ID NO:4:
   (i) SEQUENCE CHARACTERISTICS:
      (A) LENGTH:573 amino acids
      (B) TYPE:amino acid
      (D) TOPOLOGY:linear
   (ii) MOLECULE TYPE:peptide
   (xi) SEQUENCE DESCRIPTION:SEQ ID NO:4:
   Met Ala Arg Ala Val Gly Pro Glu Arg Arg Leu Leu Ala Val Tyr Thr Gly
   1               5                   10                  15
   Gly Thr Ile Gly Met Arg Ser Glu Leu Gly Val Leu Val Pro Gly Thr Gly
           20                  25                  30
   Leu Ala Ala Ile Leu Arg Thr Leu Pro Met Phe His Asp Glu Glu His Ala
   35                  40                  45                  50
   Arg Ala Arg Gly Leu Ser Glu Asp Thr Leu Val Leu Pro Pro Asp Ser Arg
               55                  60                  65
   Asn Gln Arg Ile Leu Tyr Thr Val Leu Glu Cys Gln Pro Leu Phe Asp Ser
       70                  75                  80                  85
   Ser Asp Met Thr Ile Ala Glu Trp Val Arg Val Ala Gln Thr Ile Lys Arg
                   90                  95                  100
   His Tyr Glu Gln Tyr His Gly Phe Val Val Ile His Gly Thr Asp Thr Met
           105                 110                 115
   Ala Phe Ala Ala Ser Met Leu Ser Phe Met Leu Glu Asn Leu Gln Lys Thr
   120                 125                 130                 135
   Val Ile Leu Thr Gly Ala Gln Val Pro Ile His Ala Leu Trp Ser Asp Gly
               140                 145                 150
   Arg Glu Asn Leu Leu Gly Ala Leu Leu Met Ala Gly Gln Tyr Val Ile Pro
       155                 160                 165                 170
   Glu Val Cys Leu Phe Phe Gln Asn Gln Leu Phe Arg Gly Asn Arg Ala Thr
                   175                 180                 185
   Lys Val Asp Ala Arg Arg Phe Ala Ala Phe Cys Ser Pro Asn Leu Leu Pro
           190                 195                 200
   Leu Ala Thr Val Gly Ala Asp Ile Thr Ile Asn Arg Glu Leu Val Arg Lys
   205                 210                 215                 220
   Val Asp Gly Lys Ala Gly Leu Val Val His Ser Ser Met Glu Gln Asp Val
               225                 230                 235
   Gly Leu Leu Arg Leu Tyr Pro Gly Ile Pro Ala Ala Leu Val Arg Ala Phe
       240                 245                 250                 255
   Leu Gln Pro Pro Leu Lys Gly Val Val Met Glu Thr Phe Gly Ser Gly Asn
                   260                 265                 270
   Gly Pro Thr Lys Pro Asp Leu Leu Gln Glu Leu Arg Val Ala Thr Glu Arg
           275                 280                 285
   Gly Leu Val Ile Val Asn Cys Thr His Cys Leu Gln Gly Ala Val Thr Thr
   290                 295                 300                 305
   Asp Tyr Ala Ala Gly Met Ala Met Ala Gly Ala Gly Val Ile Ser Gly Phe
               310                 315                 320
   Asp Met Thr Ser Glu Ala Ala Leu Ala Lys Leu Ser Tyr Val Leu Gly Gln
       325                 330                 335                 340
   Pro Gly Leu Ser Leu Asp Val Arg Lys Glu Leu Leu Thr Lys Asp Leu Arg
                   345                 350                 355
   Gly Glu Met Thr Pro Pro Ser Val Glu Glu Arg Arg Pro Ser Leu Gln Gly
               360                 365                 370
   Asn Thr Leu Gly Gly Gly Val Ser Trp Leu Leu Ser Leu Ser Gly Ser Gln
   375                 380                 385                 390
   Glu Ala Asp Ala Leu Arg Asn Ala Leu Val Pro Ser Leu Ala Cys Ala Ala
               395                 400                 405
   Ala His Ala Gly Asp Val Glu Ala Leu Gln Ala Leu Val Glu Leu Gly Ser
       410                 415                 420                 425
   Asp Leu Gly Leu Val Asp Phe Asn Gly Gln Thr Pro Leu His Ala Ala Ala
                   430                 435                 440
   Arg Gly Gly His Thr Glu Ala Val Thr Met Leu Leu Gln Arg Gly Val Asp
           445                 450                 455
   Val Asn Thr Arg Asp Thr Asp Gly Phe Ser Pro Leu Leu Leu Ala Val Arg
   460                 465                 470                 475
   Gly Arg His Pro Gly Val Ile Gly Leu Leu Arg Glu Ala Gly Ala Ser Leu
               480                 485                 490
   Ser Thr Gln Glu Leu Glu Glu Ala Gly Thr Glu Leu Cys Arg Leu Ala Tyr
       495                 500                 505                 510
   Arg Ala Asp Leu Glu Gly Leu Gln Val Trp Trp Gln Ala Gly Ala Asp Leu
                   515                 520                 525
   Gly Gln Pro Gly Tyr Asp Gly His Ser Ala Leu His Val Ala Glu Ala Ala
               530                 535                 540
   Gly Asn Leu Ala Val Val Ala Phe Leu Gln Ser Leu Glu Gly Ala Val Gly
   545                 550                 555                 560
   Ala Gln Ala Pro Cys Pro Glu Val Leu Pro Gly Val
               565                 570
(6) INFORMATION FOR SEQ ID NO:5:
   (i) SEQUENCE CHARACTERISTICS:
      (A) LENGTH:1695 base pairs
      (B) TYPE:nucleic acid
      (D) TOPOLOGY:linear
   (xi) SEQUENCE DESCRIPTION:SEQ ID NO:5:
   ATGGCGCGCG CATCAGGCTC CGAGAGGCAC CTGCTGCTCA TCTACACTGG CGGCACTTTG   60
   GGCATGCAGA GCAAGGGCGG GGTGCTCGTC CCCGGCCCAG GCCTGGTCAC TCTGCTGCGG  120
   ACCCTGCCCA TGTTCCATGA CAAGGAGTTC GCCCAGGCCC AGGGCCTCCC TGACCATGCT  180
   CTGGCGCTGC CCCCTGCCAG CCACGGCCCC AGGGTCCTCT ACACGGTGCT GGAGTGCCAG  240
   CCCCTCTTGG ATTCCAGCGA CATGACCATC GATGATTGGA TTCGCATAGC CAAGATCATA  300
   GAGAGGCACT ATGAGCAGTA CCAAGGCTTT GTGGTTATCC ACGGCACCGA CACCATGGCC  360
   TTTGGGGGCT CCATGCTGTC CTTCATGCTG GAAAACCTGC ACAAACCAGT CATCCTCACT  420
   GGCGCCCAGG TGCCAATCCG TGTGCTGTGG AATGACGCCC GGGAAAACCT GCTGGGGGCG  480
   TTGCTTGTGG CCGGCCAATA CATCATCCCT GAGGTCTGCC TGTTTATGAA CAGTCAGCTG  540
   TTTCGGGGAA ACCGGGTAAC CAAGGTGGAC TCCCAGAAGT TTGAGGCCTT CTGCTCCCCC  600
   AATCTGTCCC CACTAGCCAC TGTGGGCGCG GATGTCACAA TTGCCTGGGA CCTGGTGCGC  660
   AAGGTCAACT GGAAGGACCC GCTGGTGGTG CACAGCAACA TGGAGCACGA CGTGGCACTG  720
   CTGCGCCTCT ACCCTGGCAT CCCGGCCTCC CTGGTCCGGG CATTCCTGCA GCCCCCGCTC  780
   AAGGGCGTGG TCCTGGAGAC CTTCGGCTCT GGCAACGGGC CGAGCAAGCC CGACCTGCTG  840
   CAGGAGTTGC GGGCCGCGGC CCAGCGCGGC CTCATCATGG TCAACTGCAG CCAGTGCCTG  900
   CGGGGGTCTG TGACCCCGGG CTATGCCACG AGCTTGGCGG GCGCCAACAT CGTGTCCGGC  960
   TTAGACATGA CCTCAGAGGC CGCGCTGGCT AAGCTGTCCT ACGTGTTGGG CCTGCCGGAG 1020
   CTGAGCCTGG AGCGCAGGCA GGAGCTGCTG GCCAAGGATC TTCGCGGGGA AATGACACTG 1080
   CCCACGGCAG ACCTGCACCA GTCCTCTCCG CCGGGCAGCA CACTGGGGCA AGGTGTCGCC 1140
   CGGCTCTTTA GTCTGTTCGG TTGCCAGGAG GAAGATTCGG TGCAGGACGC CGTGATGCCC 1200
   AGCCTGGCCC TGGCCTTGGC CCATGCTGGT GAACTCGAGG CTCTGCAGGC ACTTATGGAG 1260
   CTGGGCAGTG ACCTGCGCCT AAAGGACTCT AATGGCCAAA CCCTGTTGCA TGTGGCTGCT 1320
   CGGAATGGGC GTGATGGCGT GGTCACCATG CTGCTGCACA GAGGCATGGA TGTCAATGCC 1380
   CGAGACCGAG ACGGCCTCAG CCCACTGCTG TTGGCTGTAC AGGGCAGGCA TCGGGAATGC 1440
   ATCAGGCTGC TGCGGAAGGC TGGGGCCTGC CTGTCCCCCC AGGACCTGAA GGATGCAGGG 1500
   ACCGAGCTGT GCAGGCTGGC ATCCAGGGCT GACATGGAAG GCCTGCAGGC ATGGGGGCAG 1560
   GCTGGGGCCG ACCTGCAGCA GCCGGGCTAT GATGGGCGCA GCGCTCTGTG TGTCGCAGAA 1620
   GCAGCCGGGA ACCAGGAGGT GCTGGCCCTT CTGCGGAACC TGGCACTTGT AGGCCCGGAA 1680
   GTGCCGCCTG CCATC                                                  1695
(7) INFORMATION FOR SEQ ID NO:6:
   (i) SEQUENCE CHARACTERISTICS:
      (A) LENGTH:1719 base pairs
      (B) TYPE:nucleic acid
      (D) TOPOLOGY:linear
   (xi) SEQUENCE DESCRIPTION:SEQ ID NO:6:
   ATGGCGCGCG CGGTGGGGCC CGAGCGGAGG CTGCTGGCCG TCTACACCGG CGGCACCATT   60
   GGCATGCGGA GTGAGCTCGG CGTGCTTGTG CCCGGGACGG GCCTGGCTGC CATCCTGAGG  120
   ACACTGCCCA TGTTCCATGA CGAGGAGCAC GCCCGAGCCC GCGGCCTCTC TGAGGACACC  180
   CTGGTGCTAC CCCCGGACAG CCGCAACCAG AGGATCCTCT ACACCGTGCT GGAGTGCCAG  240
   CCCCTCTTCG ACTCCAGTGA CATGACCATC GCTGAGTGGG TTCGCGTTGC CCAGACCATC  300
   AAGAGGCACT ACGAGCAGTA CCACGGCTTT GTGGTCATCC ACGGCACCGA CACCATGGCC  360
   TTTGCTGCCT CGATGCTGTC CTTCATGCTG GAGAACCTGC AGAAGACTGT CATCCTCACT  420
   GGGGCCCAGG TGCCCATCCA TGCCCTGTGG AGCGACGGCC GTGAGAACCT GCTGGGGGCA  480
   CTGCTCATGG CTGGCCAGTA TGTGATCCCA GAGGTCTGCC TTTTCTTCCA GAATCAGCTG  540
   TTTCGGGGCA ACCGGGCAAC CAAGGTAGAC GCTCGGAGGT TCGCAGCTTT CTGCTCCCCG  600
   AACCTGCTGC CTCTGGCCAC AGTGGGTGCT GACATCACAA TCAACAGGGA GCTGGTGCGG  660
   AAGGTGGACG GGAAGGCTGG GCTGGTGGTG CACAGCAGCA TGGAGCAGGA CGTGGGCCTG  720
   CTGCGCCTCT ACCCTGGGAT CCCTGCCGCC CTGGTTCGGG CCTTCTTGCA GCCTCCCCTG  780
   AAGGGCGTGG TCATGGAGAC CTTCGGTTCA GGGAACGGAC CCACCAAGCC CGACCTGCTG  840
   CAGGAGCTGC GGGTGGCCAC CGAGCGCGGC CTGGTCATCG TCAACTGTAC CCACTGCCTC  900
   CAGGGGGCTG TGACCACAGA CTATGCAGCT GGCATGGCCA TGGCGGGAGC CGGCGTCATC  960
   TCAGGCTTCG ACATGACATC GGAGGCCGCC CTGGCCAAGC TATCGTATGT GCTGGGCCAG 1020
   CCAGGGCTGA GCCTGGATGT CAGGAAGGAG CTGCTGACCA AGGACCTTCG GGGGGAGATG 1080
   ACGCCACCCT CGGTGGAAGA GCGCCGGCCC TCACTGCAGG GCAACACGCT GGGCGGTGGG 1140
   GTCTCCTGGC TCCTCAGTCT GAGCGGCAGC CAGGAGGCAG ATGCCCTGCG GAATGCCCTG 1200
   GTGCCCAGCC TGGCCTGTGC TGCTGCCCAC GCCGGTGACG TGGAGGCGCT GCAGGCGCTT 1260
   GTGGAGCTGG GCAGTGACCT GGGCCTGGTG GACTTTAACG GCCAAACCCC ACTGCACGCG 1320
   GCCGCCCGGG GAGGCCACAC AGAGGCAGTC ACCATGCTGC TGCAGAGAGG TGTGGACGTG 1380
   AACACCCGGG ACACGGATGG CTTCAGCCCG CTGCTGCTGG CCGTGCGGGG CAGGCATCCG 1440
   GGTGTCATTG GGTTGCTGCG GGAAGCCGGG GCCTCCCTGT CCACCCAGGA GCTGGAGGAA 1500
   GCAGGGACGG AGCTGTGCAG GCTGGCATAC AGGGCCGACC TCGAAGGCCT GCAGGTGTGG 1560
   TGGCAGGCAG GGGCTGACCT GGGGCAGCCG GGCTATGACG GGCACAGCGC CCTGCACGTC 1620
   GCAGAGGCAG CCGGGAACCT GGCAGTGGTG GCCTTTCTAC AGAGCCTGGA GGGTGCGGTT 1680
   GGTGCCCAGG CCCCATGCCC AGAAGTGCTG CCTGGTGTC                        1719
(8) INFORMATION FOR SEQ ID NO:7:
   (i) SEQUENCE CHARACTERISTICS:
      (A) LENGTH:11 amino acids
      (B) TYPE:amino acid
      (D) TOPOLOGY:linear
   (ii) MOLECULE TYPE:peptide
   (v) FRAGMENT TYPE:internal fragment
   (xi) SEQUENCE DESCRIPTION:SEQ ID NO:7:
   Leu Tyr Pro Gly Ile Pro Ala Ser Leu Val Arg
   1               5                   10
(9) INFORMATION FOR SEQ ID NO:8:
   (i) SEQUENCE CHARACTERISTICS:
      (A) LENGTH:16 amino acids
      (B) TYPE:amino acid
      (D) TOPOLOGY:linear
   (ii) MOLECULE TYPE:peptide
   (v) FRAGMENT TYPE:internal fragment
   (xi) SEQUENCE DESCRIPTION:SEQ ID NO:8:
   His Leu Leu Leu Ile Tyr Thr Gly Gly Thr Leu Gly Met Gln Ser Lys
   1               5                   10                  15
(10) INFORMATION FOR SEQ ID NO:9:
   (i) SEQUENCE CHARACTERISTICS:
      (A) LENGTH:9 amino acids
      (B) TYPE:amino acid
      (D) TOPOLOGY:linear
   (ii) MOLECULE TYPE:peptide
   (v) FRAGMENT TYPE:internal fragment
   (xi) SEQUENCE DESCRIPTION:SEQ ID NO:9:
   Ser Phe Met Leu Glu Asn Leu His Lys
   1               5
(11) INFORMATION FOR SEQ ID NO:10:
   (i) SEQUENCE CHARACTERISTICS:
      (A) LENGTH:1928 base pairs
      (B) TYPE:nucleic acid
      (C) STRANDEDNESS:double
      (D) TOPOLOGY:linear
   (ii) MOLECULE TYPE:cDNA to mRNA
   (iii) HYPOTHETICAL:No
   (iv) ANTISENSE:No
   (vi)ORIGINAL SOURCE:
      (A) ORGANISM:guinea pig
      (F) TISSUE TYPE:liver
   (ix) FEATURE:
      (A1) NAME/KEY:1-19 5'-UTR
      (C1) IDENTIFICATION METHOD:S
      (A2) NAME/KEY:20-1714 mat peptide
      (C2) IDENTIFICATION METHOD:S
      (A3) NAME/KEY:1715-1928 3'-UTR
      (C3) IDENTIFICATION METHOD:S
   (xi) SEQUENCE DESCRIPTION:SEQ ID NO:10:
   GAGTGGCTTA GCCGCAGGC                                                19
   ATG GCG CGC GCA TCA GGC TCC GAG AGG CAC CTG CTG CTC ATC TAC ACT     67
   Met Ala Arg Ala Ser Gly Ser Glu Arg His Leu Leu Leu Ile Tyr Thr
   1               5                   10                  15
   GGC GGC ACT TTG GGC ATG CAG AGC AAG GGC GGG GTG CTC GTC CCC GGC    115
   Gly Gly Thr Leu Gly Met Gln Ser Lys Gly Gly Val Leu Val Pro Gly
               20                  25                  30
   CCA GGC CTG GTC ACT CTG CTG CGG ACC CTG CCC ATG TTC CAT GAC AAG    163
   Pro Gly Leu Val Thr Leu Leu Arg Thr Leu Pro Met Phe His Asp Lys
           35                  40                  45
   GAG TTC GCC CAG GCC CAG GGC CTC CCT GAC CAT GCT CTG GCG CTG CCC   211
   Glu Phe Ala Gln Ala Gln Gly Leu Pro Asp His Ala Leu Ala Leu Pro
       50                  55                  60
   CCT GCC AGC CAC GGC CCC AGG GTC CTC TAC ACG GTG CTG GAG TGC CAG   259
   Pro Ala Ser His Gly Pro Arg Val Leu Tyr Thr Val Leu Glu Cys Gln
   65                  70                  75                  80
   CCC CTC TTG GAT TCC AGC GAC ATG ACC ATC GAT GAT TGG ATT CGC ATA   307
   Pro Leu Leu Asp Ser Ser Asp Met Thr Ile Asp Asp Trp Ile Arg Ile
                   85                  90                  95
   GCC AAG ATC ATA GAG AGG CAC TAT GAG CAG TAC CAA GGC TTT GTG GTT   355
   Ala Lys Ile Ile Glu Arg His Tyr Glu Gln Tyr Gln Gly Phe Val Val
               100                 105                 110
   ATC CAC GGC ACC GAC ACC ATG GCC TTT GGG GGC TCC ATG CTG TCC TTC   403
   Ile His Gly Thr Asp Thr Met Ala Phe Gly Ala Ser Met Leu Ser Phe
           115                 120                 125
   ATG CTG GAA AAC CTG CAC AAA CCA GTC ATC CTC ACT GGC GCC CAG GTG   451
   Met Leu Glu Asn Leu His Lys Pro Val Ile Leu Thr Gly Ala Gln Val
       130                 135                 140
   CCA ATC CGT GTG CTG TGG AAT GAC GCC CGG GAA AAC CTG CTG GGG GCG   499
   Pro Ile Arg Val Leu Trp Asn Asp Ala Arg Glu Asn Leu Leu Gly Ala
   145                 150                 155                 160
   TTG CTT GTG GCC GGC CAA TAC ATC ATC CCT GAG GTC TGC CTG TTT ATG   547
   Leu Leu Val Ala Gly Gln Tyr Ile Ile Pro Glu Val Cys Leu Phe Met
                   165                 170                 175
   AAC AGT CAG CTG TTT CGG GGA AAC CGG GTA ACC AAG GTG GAC TCC CAG   595
   Asn Ser Gln Leu Phe Arg Gly Asn Arg Val Thr Lys Val Asp Ser Gln
               180                 185                 190
   AAG TTT GAG GCC TTC TGC TCC CCC AAT CTG TCC CCA CTA GCC ACT GTG   643
   Lys Phe Glu Ala Phe Cys Ser Pro Asn Leu Ser Pro Leu Ala Thr Val
           195                 200                 205
   GGC GCG GAT GTC ACA ATT GCC TGG GAC CTG GTG CGC AAG GTC AAC TGG   691
   Gly Ala Asp Val Thr Ile Ala Trp Asp Leu Val Arg Lys Val Asn Trp
       215                 220                 225
   AAG GAC CCG CTG GTG GTG CAC AGC AAC ATG GAG CAC GAC GTG GCA CTG   739
   Lys Asp Pro Leu Val Val His Ser Asn Met Glu His Asp Val Ala Leu
   225                 230                 235                 240
   CTG CGC CTC TAC CCT GGC ATC CCG GCC TCC CTG GTC CGG GCA TTC CTG   787
   Leu Arg Leu Tyr Pro Gly Ile Pro Ala Ser Leu Val Arg Ala Phe Leu
                   245                 250                 255
   CAG CCC CCG CTC AAG GGC GTG GTC CTG GAG ACC TTC GGC TCT GGC AAC   835
   Gln Pro Pro Leu Lys Gly Val Val Leu Glu Thr Phe Gly Ser Gly Asn
               260                 265                 270
   GGG CCG AGC AAG CCC GAC CTG CTG CAG GAG TTG CGG GCC GCG GCC CAG   883
   Gly Pro Ser Lys Pro Asp Leu Leu Gln Glu Leu Arg Ala Ala Ala Gln
           275                 280                 285
   CGC GGC CTC ATC ATG GTC AAC TGC AGC CAG TGC CTG CGG GGG TCT GTG   931
   Arg Gly Leu Ile Met Val Asn Cys Ser Gln Cys Leu Arg Gly Ser Val
       290                 295                 300
   ACC CCG GGC TAT GCC ACG AGC TTG GCG GGC GCC AAC ATC GTG TCC GGC   979
   Thr Pro Gly Tyr Ala Thr Ser Leu Ala Gly Ala Asn Ile Val Ser Gly
   305                 310                 315                 320
   TTA GAC ATG ACC TCA GAG GCC GCG CTG GCT AAG CTG TCC TAC GTG TTG  1027
   Leu Asp Met Thr Ser Glu Ala Ala Leu Ala Lys Leu Ser Tyr Val Leu
                   325                 330                 335
   GGC CTG CCG GAG CTG AGC CTG GAG CGC AGG CAG GAG CTG CTG GCC AAG  1075
   Gly Leu Pro Glu Leu Ser Leu Glu Arg Arg Gln Glu Leu Leu Ala Lys
               340                 345                 350
   GAT CTT CGC GGG GAA ATG ACA CTG CCC ACG GCA GAC CTG CAC CAG TCC  1123
   Asp Leu Arg Gly Glu Met Thr Leu Pro Thr Ala Asp Leu His Gln Ser
           355                 360                 365
   TCT CCG CCG GGC AGC ACA CTG GGG CAA GGT GTC GCC CGG CTC TTT AGT  1171
   Ser Pro Pro Gly Ser Thr Leu Gly Gln Gly Val Ala Arg Leu Phe Ser
       370                 375                 380
   CTG TTC GGT TGC CAG GAG GAA GAT TCG GTG CAG GAC GCC GTG ATG CCC  1219
   Leu Phe Gly Cys Gln Glu Glu Asp Ser Val Gln Asp Ala Val Met Pro
   385                 390                 395                 400
   AGC CTG GCC CTG GCC TTG GCC CAT GCT GGT GAA CTC GAG GCT CTG CAG  1267
   Ser Leu Ala Leu Ala Leu Ala His Ala Gly Glu Leu Glu Ala Leu Gln
                   405                 410                 415
   GCA CTT ATG GAG CTG GGC AGT GAC CTG CGC CTA AAG GAC TCT AAT GGC  1315
   Ala Leu Met Glu Leu Gly Ser Asp Leu Arg Leu Lys Asp Ser Asn Gly
               420                 425                 430
   CAA ACC CTG TTG CAT GTG GCT GCT CGG AAT GGG CGT GAT GGC GTG GTC  1363
   Gln Thr Leu Leu His Val Ala Ala Arg Asn Gly Arg Asp Gly Val Val
           435                 440                 445
   ACC ATG CTG CTG CAC AGA GGC ATG GAT GTC AAT GCC CGA GAC CGA GAC  1411
   Thr Met Leu Leu His Arg Gly Met Asp Val Asn Ala Arg Asp Arg Asp
       450                 455                 460
   GGC CTC AGC CCA CTG CTG TTG GCT GTA CAG GGC AGG CAT CGG GAA TGC  1459
   Gly Leu Ser Pro Leu Leu Leu Ala Val Gln Gly Arg His Arg Glu Cys
   465                 470                 475                 480
   ATC AGG CTG CTG CGG AAG GCT GGG GCC TGC CTG TCC CCC CAG GAC CTG  1507
   Ile Arg Leu Leu Arg Lys Ala Gly Ala Cys Leu Ser Pro Gln Asp Leu
                   485                 490                 495
   AAG GAT GCA GGG ACC GAG CTG TGC AGG CTG GCA TCC AGG GCT GAC ATG  1555
   Lys Asp Ala Gly Thr Glu Leu Cys Arg Leu Ala Ser Arg Ala Asp Met
               500                 505                 510
   GAA GGC CTG CAG GCA TGG GGG CAG GCT GGG GCC GAC CTG CAG CAG CCG  1603
   Glu Gly Leu Gln Ala Trp Gly Gln Ala Gly Ala Asp Leu Gln Gln Pro
           515                 520                 525
   GGC TAT GAT GGG CGC AGC GCT CTG TGT GTC GCA GAA GCA GCC GGG AAC  1651
   Gly Tyr Asp Gly Arg Ser Ala Leu Cys Val Ala Glu Ala Ala Gly Asn
       530                 535                 540
   CAG GAG GTG CTG GCC CTT CTG CGG AAC CTG GCA CTT GTA GGC CCG GAA  1699
   Gln Glu Val Leu Ala Leu Leu Arg Asn Leu Ala Leu Val Gly Pro Glu
   545                 550                 555                 560
   GTG CCG CCT GCC ATC T GATCGCCAGC AATCCCGCTG TGGTGTGAGC CACTCCGCCA 1755
   Val Pro Pro Ala Ile
                   565
   TCTGCTGCTT TGACCCACTC GAGGGACCCT AGCACACGAC CCCCCAGCAG GATGCACCCC 1815
   ACTACTTAGA GTATACCCCA GGCTGGCTCA GTGACAAGCT GCAAAGGTCT TTGTTGGCAG 1875
   AACAGCAATA AAGTAACTAC AGAGTGGCCA AAAAAAAAAA AAAAAAAAAA AAA        1928
(12) INFORMATION FOR SEQ ID NO:11:
   (i) SEQUENCE CHARACTERISTICS:
      (A) LENGTH:2096 base pairs
      (B) TYPE:nucleic acid
      (C) STRANDEDNESS:double
      (D) TOPOLOGY:linear
   (ii) MOLECULE TYPE:cDNA to mRNA
   (iii) HYPOTHETICAL:No
   (iv) ANTISENSE:No
   (vi) ORIGINAL SOURCE:
      (A) ORGANISM:human
      (F) TISSUE TYPE:liver
   (ix)FEATURE:
      (A1) NAME/KEY:1-92 5'-UTR
      (C1) IDENTIFICATION METHOD:S
      (A2) NAME/KEY:93-1811 mat peptide
      (C2) IDENTIFICATION METHOD:S
      (A4) NAME/KEY:1812-2096 3'-UTR
      (C4) IDENTIFICATION METHOD:S
   (xi) SEQUENCE DESCRIPTION:SEQ ID NO:11:
   CGCCCCGGGC CTCCTCCGCG CAGTCCCTGA GTCCCGCAGG CCCTGCGTCC CCGCTGCACA   60
   CCCCCGTCCA CTCCCGTGGT CCCCGGTCCG GC                                 92
   ATG GCG CGC GCG GTG GGG CCC GAG CGG AGG CTG CTG GCC GTC TAC ACC    140
   Met Ala Arg Ala Val Gly Pro Glu Arg Arg Leu Leu Ala Val Tyr Thr
   1               5                   10                  15
   GGC GGC ACC ATT GGC ATG CGG AGT GAG CTC GGC GTG CTT GTG CCC GGG    188
   Gly Gly Thr Ile Gly Met Arg Ser Glu Leu Gly Val Leu Val Pro Gly
               20                  25                  30
   ACG GGC CTG GCT GCC ATC CTG AGG ACA CTG CCC ATG TTC CAT GAC GAG    236
   Thr Gly Leu Ala Ala Ile Leu Arg Thr Leu Pro Met Phe His Asp Glu
           35                  40                  45
   GAG CAC GCC CGA GCC CGC GGC CTC TCT GAG GAC ACC CTG GTG CTA CCC    284
   Glu His Ala Arg Ala Arg Gly Leu Ser Glu Asp Thr Leu Val Leu Pro
       50                  55                  60
   CCG GAC AGC CGC AAC CAG AGG ATC CTC TAC ACC GTG CTG GAG TGC CAG    332
   Pro Asp Ser Arg Asn Gln Arg Ile Leu Tyr Thr Val Leu Glu Cys Gln
   65                  70                  75                  80
   CCC CTC TTC GAC TCC AGT GAC ATG ACC ATC GCT GAG TGG GTT CGC GTT    380
   Pro Leu Phe Asp Ser Ser Asp Met Thr Ile Ala Glu Trp Val Arg Val
                   85                  90                  95
   GCC CAG ACC ATC AAG AGG CAC TAC GAG CAG TAC CAC GGC TTT GTG GTC    428
   Ala Gln Thr Ile Lys Arg His Tyr Glu Gln Tyr His Gly Phe Val Val
               100                 105                 110
   ATC CAC GGC ACC GAC ACC ATG GCC TTT GCT GCC TCG ATG CTG TCC TTC    476
   Ile His Gly Thr Asp Thr Met Ala Phe Ala Ala Ser Met Leu Ser Phe
           115                 120                 125
   ATG CTG GAG AAC CTG CAG AAG ACT GTC ATC CTC ACT GGG GCC CAG GTG    524
   Met Leu Glu Asn Leu Gln Lys Thr Val Ile Leu Thr Gly Ala Gln Val
       130                 135                 140
   CCC ATC CAT GCC CTG TGG AGC GAC GGC CGT GAG AAC CTG CTG GGG GCA    572
   Pro Ile His Ala Leu Trp Ser Asp Gly Arg Glu Asn Leu Leu Gly Ala
   145                 150                 155                 160
   CTG CTC ATG GCT GGC CAG TAT GTG ATC CCA GAG GTC TGC CTT TTC TTC    620
   Leu Leu Met Ala Gly Gln Tyr Val Ile Pro Glu Val Cys Leu Phe Phe
                   165                 170                 175
   CAG AAT CAG CTG TTT CGG GGC AAC CGG GCA ACC AAG GTA GAC GCT CGG    668
   Gln Asn Gln Leu Phe Arg Gly Asn Arg Ala Thr Lys Val Asp Ala Arg
               180                 185                 190
   AGG TTC GCA GCT TTC TGC TCC CCG AAC CTG CTG CCT CTG GCC ACA GTG    716
   Arg Phe Ala Ala Phe Cys Ser Pro Asn Leu Leu Pro Leu Ala Thr Val
           195                 200                 205
   GGT GCT GAC ATC ACA ATC AAC AGG GAG CTG GTG CGG AAG GTG GAC GGG    764
   Gly Ala Asp Ile Thr Ile Asn Arg Glu Leu Val Arg Lys Val Asp Gly
       210                 215                 220
   AAG GCT GGG CTG GTG GTG CAC AGC AGC ATG GAG CAG GAC GTG GGC CTG    812
   Lys Ala Gly Leu Val Val His Ser Ser Met Glu Gln Asp Val Gly Leu
   225                 230                 235                 240
   CTG CGC CTC TAC CCT GGG ATC CCT GCC GCC CTG GTT CGG GCC TTC TTG    860
   Leu Arg Leu Tyr Pro Gly Ile Pro Ala Ala Leu Val Arg Ala Phe Leu
                   245                 250                 255
   CAG CCT CCC CTG AAG GGC GTG GTC ATG GAG ACC TTC GGT TCA GGG AAC    908
   Gln Pro Pro Leu Lys Gly Val Val Met Glu Thr Phe Gly Ser Gly Asn
               260                 265                 270
   GGA CCC ACC AAG CCC GAC CTG CTG CAG GAG CTG CGG GTG GCC ACC GAG    956
   Gly Pro Thr Lys Pro Asp Leu Leu Gln Glu Leu Arg Val Ala Thr Glu
           275                 280                 285
   CGC GGC CTG GTC ATC GTC AAC TGT ACC CAC TGC CTC CAG GGG GCT GTG   1004
   Arg Gly Leu Val Ile Val Asn Cys Thr His Cys Leu Gln Gly Ala Val
       290                 295                 300
   ACC ACA GAC TAT GCA GCT GGC ATG GCC ATG GCG GGA GCC GGC GTC ATC   1052
   Thr Thr Asp Tyr Ala Ala Gly Met Ala Met Ala Gly Ala Gly Val Ile
   305                 310                 315                 320
   TCA GGC TTC GAC ATG ACA TCG GAG GCC GCC CTG GCC AAG CTA TCG TAT   1100
   Ser Gly Phe Asp Met Thr Ser Glu Ala Ala Leu Ala Lys Leu Ser Tyr
                   325                 330                 335
   GTG CTG GGC CAG CCA GGG CTG AGC CTG GAT GTC AGG AAG GAG CTG CTG   1148
   Val Leu Gly Gln Pro Gly Leu Ser Leu Asp Val Arg Lys Glu Leu Leu
               340                 345                 350
   ACC AAG GAC CTT CGG GGG GAG ATG ACG CCA CCC TCG GTG GAA GAG CGC   1196
   Thr Lys Asp Leu Arg Gly Glu Met Thr Pro Pro Ser Val Glu Glu Arg
           355                 360                 365
   CGG CCC TCA CTG CAG GGC AAC ACG CTG GGC GGT GGG GTC TCC TGG CTC   1244
   Arg Pro Ser Leu Gln Gly Asn Thr Leu Gly Gly Gly Val Ser Trp Leu
       370                 375                 380
   CTC AGT CTG AGC GGC AGC CAG GAG GCA GAT GCC CTG CGG AAT GCC CTG   1292
   Leu Ser Leu Ser Gly Ser Gln Glu Ala Asp Ala Leu Arg Asn Ala Leu
   385                 390                 395                 400
   GTG CCC AGC CTG GCC TGT GCT GCT GCC CAC GCC GGT GAC GTG GAG GCG   1340
   Val Pro Ser Leu Ala Cys Ala Ala Ala His Ala Gly Asp Val Glu Ala
                   405                 410                 415
   CTG CAG GCG CTT GTG GAG CTG GGC AGT GAC CTG GGC CTG GTG GAC TTT   1388
   Leu Gln Ala Leu Val Glu Leu Gly Ser Asp Leu Gly Leu Val Asp Phe
               420                 425                 430
   AAC GGC CAA ACC CCA CTG CAC GCG GCC GCC CGG GGA GGC CAC ACA GAG   1436
   Asn Gly Gln Thr Pro Leu His Ala Ala Ala Arg Gly Gly His Thr Glu
           435                 440                 445
   GCA GTC ACC ATG CTG CTG CAG AGA GGT GTG GAC GTG AAC ACC CGG GAC   1484
   Ala Val Thr Met Leu Leu Gln Arg Gly Val Asp Val Asn Thr Arg Asp
       450                 455                 460
   ACG GAT GGC TTC AGC CCG CTG CTG CTG GCC GTG CGG GGC AGG CAT CCG   1532
   Thr Asp Gly Phe Ser Pro Leu Leu Leu Ala Val Arg Gly Arg His Pro
   465                 470                 475                 480
   GGT GTC ATT GGG TTG CTG CGG GAA GCC GGG GCC TCC CTG TCC ACC CAG   1580
   Gly Val Ile Gly Leu Leu Arg Glu Ala Gly Ala Ser Leu Ser Thr Gln
                   485                 490                 495
   GAG CTG GAG GAA GCA GGG ACG GAG CTG TGC AGG CTG GCA TAC AGG GCC   1628
   Glu Leu Glu Glu Ala Gly Thr Glu Leu Cys Arg Leu Ala Tyr Arg Ala
               500                 505                 510
   GAC CTC GAA GGC CTG CAG GTG TGG TGG CAG GCA GGG GCT GAC CTG GGG   1676
   Asp Leu Glu Gly Leu Gln Val Trp Trp Gln Ala Gly Ala Asp Leu Gly
           515                 520                 525
   CAG CCG GGC TAT GAC GGG CAC AGC GCC CTG CAC GTC GCA GAG GCA GCC   1724
   Gln Pro Gly Tyr Asp Gly His Ser Ala Leu His Val Ala Glu Ala Ala
       530                 535                 540
   GGG AAC CTG GCA GTG GTG GCC TTT CTA CAG AGC CTG GAG GGT GCG GTT   1772
   Gly Asn Leu Ala Val Val Ala Phe Leu Gln Ser Leu Glu Gly Ala Val
   545                 550                 555                 560
   GGT GCC CAG GCC CCA TGC CCA GAA GTG CTG CCT GGT GTC TA ACCTGAAGGC 1823
   Gly Ala Gln Ala Pro Cys Pro Glu Val Leu Pro Gly Val
                   565                 570
   GTCCTGCTGC AGTATAAGCC ATTCCTTCCT CCCATGACCT GCTGGAGGGG TCTCAGGCAT 1883
   GACCCCACTG CTGGGGCTGC TTCCCAGCCT GCTCTCATGT AAAGCCTGAA GGCCTTTGTT 1943
   GGGCAGGACG GCAATAAAGT CTCTGACATC CCCTCACCAG GTCTGTACAG CCTGGCTCTG 2003
   AGAGGCTCTG TCTGGGTCCG GGACTGTGAA AAAAAAAAAA AAAAAAAAAA AAAAAAAAAA 2063
   AAAAAAAAAA AAAAAAAAAA AAAAAAAAAA AAA                              2096

## Description

The present invention relates to a novel DNA which codes for L-asparaginase, more particularly, to a DNA which codes for a mammalian L-asparaginase.

L-Asparaginase (EC 3.5.1.1), an amidohydrolase which releases L-aspartic acid and ammonia when acts on L-asparagine, is an enzyme which plays a major role in the metabolism of L-asparagine in plants, animals and microorganisms, and it has been energetically studied on its actual use as an antitumor agent since John G. Kidd had reported the inhibitory activity of L-asparaginase on lymphoma in "*The Journal of Experimental* Medicine", Vol.98, pp.565-583 (1953). As a result, an L-asparaginase from *Escherichia coli* is now used as a therapeutic agent for leukemia and lymphoma.

However, the aforesaid L-asparaginase with a satisfactory antitumor activity is only an external protein for humans, and the administration of conventional compositions containing the L-asparaginase to patients frequently causes serious side effects such as hyperergy including anaphylaxis shock, urticaria, edema, wheeze and dyspnea. Therefore, these compositions are inevitably restricted their administration doses and frequencies by a large margin, and some proposals to reduce or even diminish such side effects have been made.

As a first proposal, Japanese Patent Laid-Open No.119,082/79 discloses an L-asparaginase from *Escherichia coli* which is chemically modified by blocking at least 65% amino acids of the L-asparaginase with 2-O- substituted polyethylene glycol-4,6-dichloro-S-triazine. As a second proposal, Japanese Patent Laid-Open No.320,684/92 discloses a human L-asparaginase which is obtained from a culture of fibroblasts from human lung, stomach or breast. The first proposal has a merit that it can use an L-asparaginase from *Escherichia coli* which is readily preparable on an industrial scale, and has a demerit that the control of the modification reaction is difficult and the side effects could not be eliminated. The second proposal has a merit that, unlike the L-asparaginase from *Escherichia coli*, human L-asparaginase does not substantially induce antibodies even when administered to patients, and has a demerit that the productivity of human fibroblasts is not sufficient as disclosed in Japanese Patent Laid-Open No.320,684/92, so that a relatively large-scale cultivation is inevitable to obtain a satisfactory amount of L-asparaginase.

Recently, recombinant DNA technology has made a remarkable progress. Now, even a polypeptide with an incomplete elucidation of its amino acid sequence can be readily prepared in a desired amount, if only a gene coding for the polypeptide is once isolated and decoded for base sequence, by preparing a recombinant DNA having a DNA which codes for the polypeptide, introducing the DNA into microorganisms or cells of animals or plants to obtain transformants, and culturing the transformants.

In view of the foregoing, a gene coding for a mammalian L-asparaginase, preferably, the isolation of a gene coding for human L-asparaginase and the prompt elucidation of its base sequence have been in great demand in this field.

The present invention provides a DNA coding for mammalian L-asparaginase, which is characterised in that said DNA comprises a base sequence which encodes both amino acid sequences as shown in SEQ ID NO: 1 and SEQ ID NO: 2 as well as that said DNA is hybridizable with either or both DNA comprising a base sequence as shown in SEQ ID NO: 5 or DNA comprising a base sequence as shown in SEQ ID NO: 6 at 50°C in a solution of 5 x SSPE, 5 x Denhardt's solution, 0.5 w/v % SDS, and 100 µg/ml of a denatured salmon sperm.

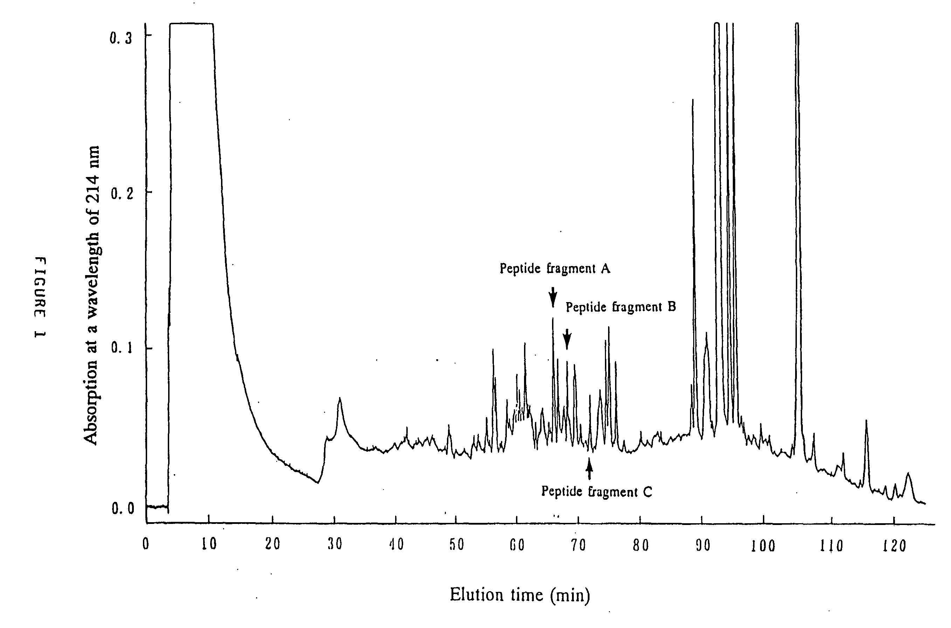

The present invention will now be described in further detail, by way of example only, with reference to the accompanying drawings, in which:-
FIG 1 is a peptide map of a guinea pig L-asparaginase.

The DNA according to the present invention expresses the production of a mammalian L-asparaginase having a specific amino acid sequence by introducing it into cells or microorganisms to form transformants.

This invention was made based on the finding of a novel DNA which codes for a guinea pig L-asparaginase. Although the existence of L-asparaginase in guinea pigs' sera has been known, the property and feature has not been substantially revealed.

The present inventors isolated a substance with an L-asparaginase activity from a guinea pig serum by using purification methods comprising column chromatography as a main technique, and determined its partial amino acid sequence. Based on the sequence, a primer was chemically synthesized and subjected to the RT-PCR reaction using as a template a mRNA, collected from liver cells of the guinea pig, to obtain a DNA fragment which partially codes for the L-asparaginase. The present inventors used the fragment as a probe and energetically screened cDNA libraries prepared from the mRNA to obtain a DNA fragment having the base sequence of SEQ ID NO:5 consisting of 1,695 base pairs. The decoding of the base sequence revealed that the guinea pig L-asparaginase consists of 565 amino acids and has the amino acid sequence of SEQ ID NO:3.

With these findings, the present inventors continued studying mRNAs collected from human liver cells and have succeeded in isolating a gene which codes for human L-asparaginase. The gene has the base sequence of SEQ ID NO:6 which consists of 1,719 base pairs, and the decoding revealed that the gene encodes the amino acids sequence consisting of 573 amino acids in SEQ ID NO:4.

The techniques used to reveal these amino acid sequences and base sequences of SEQ ID Nos:3-6 are summarized below:
(1) An L-asparaginase was isolated from a guinea pig serum and highly purified by purification methods comprising chromatography as a main technique;
(2) The L-asparaginase was trypsinized, and from the resulting mixture were isolated three peptide fragments which were then determined for amino acid sequence;
(3) A mRNA was collected from the guinea pig liver cells. By using the mRNA as a template, it was subjected to the RT-PCR reaction in the presence of a primer, which had been chemically synthesized based on the above amino acid sequences, to obtain DNA fragments which were then screened by a probe that had been chemically synthesized based on those amino acid sequences. Thus, a DNA fragment, which partially encodes the active site of the guinea pig L-asparaginase, was obtained;
(4) After labeling the DNA fragment, it was hybridized with a cDNA library, prepared by using the mRNA as a template, followed by selecting transformants which showed a strong hybridization;
(5) From the transformants a cDNA was collected, determined for base sequence, and decoded for amino acid sequence. Comparison of the decoded amino acid sequence to the above partial amino acid sequences revealed that the guinea pig L-asparaginase has the amino acid sequence of SEQ ID NO:3 and is encoded by the base sequence of SEQ ID NO:5;
(6) A cDNA library was prepared by using a mRNA collected from human liver cells, and a DNA fragment having the base sequence of SEQ ID NO:5 was prepared, then labeled and hybridized with the cDNA library, followed by selecting transformants which showed a strong hybridization; and
(7) From the transformants a cDNA was collected, determined for base sequence, and decoded for amino acid sequence, revealing that human L-asparaginase has the amino acid sequence of SEQ ID NO:4 and is encoded by the base sequence of SEQ ID NO:6.

Comparing the amino acid sequences of SEQ ID NOs:3 and 4, they have a common amino acid sequence of SEQ ID NO:1 or 2. The amino acid sequence of SEQ ID NO:1 corresponds to a partial amino acid sequence consisting of amino acids 16-19 in SEQ ID NO:3 or 4, while the amino acid sequence of SEQ ID NO:2 corresponds to a partial amino acid sequence consisting of amino acids 114-118 in SEQ ID NO:3 or 4. Because of these commonality, the mammalian L-asparaginases as referred to in the present invention include those which have the amino acid sequence of SEQ ID NO:1 or 2. As representative examples, a guinea pig L-asparaginase with the amino acid sequence of SEQ ID NO:3, a human L-asparaginase with the amino acid sequence of SEQ ID NO:4, and their variants complemental to these amino acid sequences can be mentioned. These variants include those which one or more amino acids in SEQ ID NO:3 or 4 are replaced with other amino acids, those which one or more other amino acids are added to the N- and/or C-terminals of the amino acid sequence in SEQ ID NO:3 or 4, and those which one or more amino acids in the N- and/or C-terminals of the amino acid sequence in SEQ ID NO:3 or 4 are deleted while retaining the amino acid sequence of SEQ ID NO:1 or 2 and without substantially losing an L-asparaginase activity.

The DNAs according to the present invention mean those which encode the aforesaid amino acid sequences. Examples of these DNAs are those which have the base sequences of SEQ ID NOs:6, 10 and 11, and those which have homologous and complementary base sequences to these base sequences of SEQ ID NOs:6, 10 and 11. These base sequences may be changed their one or more bases with other bases by means of the degeneracy of genetic code without alternating amino acid sequences they encode.

To allow the present DNAs to actually express the production of mammalian L-asparaginases in hosts, one or more bases of the base sequences in the DNAs, which code for the present L-asparaginases and their variants, can be replaced with other bases.

Any natural or artificial DNAs can be used as the present DNAs as long as they have any one of those base sequences. The natural sources for the present DNAs are, for example, livers of guinea pigs and humans. From these liver cells, DNAs having the base sequence of SEQ ID NO:5 or 6 can be obtained. To artificially synthesize the present DNAs, they can be chemically synthesized based on the base sequence of SEQ ID NO:5 or 6, or prepared by inserting DNAs coding for the amino acid sequence of SEQ ID NO:3 or 4 into self-replicable vectors to obtain recombinant DNAs, introducing the recombinant DNAs into appropriate hosts to obtain transformants, culturing the transformants, separating the proliferated cells from the cultures, and collecting from the cells the objective plasmids containing the present DNAs.

These DNAs thus obtained can be introduced into transformants by conventional methods. The transformants thus obtained produce mammalian L-asparaginases intra- or extracellularly when cultured. Unlike conventional L-asparaginases produced form *Escherichia coli,* mammalian L-asparaginases have a merit that they do not substantially cause side effects such as anaphylaxis shock and hyperergy even when administered to patients repeatedly. These mammalian L-asparaginases can be, therefore, selectively used alone or in combination with other drugs to treat acute leukemia, acute lymphocytic leukemia, and mammalian malignant tumors in general including malignant tumors.

The present invention is explained with reference to the following Examples, and the techniques used therein are well known in the art: Examples of such are those described in "*Molecular Cloning A Laboratory Manual, 2nd Edition*" by T. Maniatis et al., published by Cold Spring Harbor Laboratory Press, New York, USA (1989), and in *"Laboratory Manual for Genetic Engineering*" by Masami MURAMATSU, published by Maruzen Co., Ltd., Tokyo, Japan (1988).

### Example 1

### Purification of guinea pig L-asparaginase

One hundred guinea pigs, 8-10-week-old, were collected their blood which were then pooled and treated in a conventional manner to obtain a 300 ml serum. Ammonium sulfate was added to the serum to give a saturation degree of 50 w/v % , allowed to stand at 4°C for 2 hours, and centrifuged at about 8, 000 rpm for 30 min. The precipitate was collected, dissolved in 10 mM phosphate buffer (pH 7.0), and dialyzed against a fresh preparation of the same buffer at 4°C for 18 hours. The dialyzed inner solution was fed to a column packed with 300 ml of **"SEPHACRYL S-300",** a gel for gel filtration column chromatography commercialized by Pharmacia LKB Biotechnology AB, Uppsala, Sweden, which had been equilibrated with a fresh preparation of the same buffer, followed by feeding to the column a fresh preparation of the same buffer.

Fractions corresponding to a molecular weight of about 150,000 daltons were collected, pooled, fed to a column packed with "**DEAE-5PW**", a gel for high-performance liquid chromatography commercialized by Tosoh Corporation Tokyo, Japan, which had been equilibrated with 20 mM phosphate buffer (pH 7.0), followed by washing the column with a fresh preparation of the same buffer and feeding to the column at a flow rate of 13 ml/min a linear gradient buffer of sodium chloride increasing from 0 M to 1 M in 20 mM phosphate buffer (pH 7.0). About 120 ml fractions eluted at a concentration of about 0.25 M of sodium chloride were collected, pooled and dialyzed against distilled water at 4°C for 4 hours. The dialyzed inner solution was fed to a column packed with 100 ml **"BLUE SEPHAROSE CL-6B"**, a gel for affinity chromatography commercialized by Pharmacia LKB Biotechnology AB, Uppsala, Sweden, followed by feeding to the column with a fresh preparation of the same buffer to elute a guinea pig L-asparaginase.

Fractions containing the L-asparaginase were collected, pooled and concentrated, and the concentrate was fed to a column packed with **"HILOARD SUPERDEX 200"**, a gel for gel filtration chromatography commercialized by Pharmacia LKB Biotechnology AB, Uppsala, Sweden, followed by feeding to the column with a fresh preparation of the same buffer. Fractions corresponding to a molecular weight of about 150,000 daltons were collected, pooled and concentrated to obtain about 400 µg of the guinea pig L-asparaginase with a specific activity of about 10 units/mg protein per guinea pig.

Throughout the present specification, the activity of L-asparaginase is assayed as follows and expressed with units: Distribute a sample to a 96-well microplate in a volume of 160 µl/well, and add to each well a 40 µl solution containing 1.4 mg/ml of L-asparagine dissolved in 50 mM phosphate buffer (pH 7.0). Incubate the microplate at 37°C for 10-30 min and quantify the released L-aspartic acid by an amino acid analyzer. Provide a system using an L-asparaginase from *Escherichia coli* which had been diluted to give a concentration of 1.0, 0.5 or 0.25 units/ml, and similarly as above treat the system and quantify the released L-aspartic acid. Prot the measurements to draw a working line. To estimate the activity of the sample, plot the L-aspartic acid content in the sample system. One unit activity of L-asparaginase is defined as the amount of a polypeptide having an L-asparaginase activity that releases one µmol ammonia from L-asparagine per min when allowed to react under the above conditions.

### Example 2

### Partial amino acid sequence of quinea pig L-asparaginase

A portion of the aqueous solution containing the purified L-asparaginase in Example 1 was placed in a container and concentrated up to give about 50 µl. To the concentrate was added 25 µl of a mixture solution consisting of 3 w/v % sodium dodecyl sulfate (SDS), 60 w/v % glycerol and 60 mg/ml dithiothreitol, and the resulting solution was incubated at 50°C for 30 min, transferred onto 10 w/v % polyacrylamide gels, and electrophoresed in a conventional manner. The resulting gels were successively soaked for staining in 50 v/v % aqueous methanol containing 0.1 w/v % Coomassie Brilliant Blue R250 and 10 v/v % acetic acid, destained by washing repeatedly with 12 v/v % aqueous methanol containing 7 v/v % acetic acid, and soaked in distilled water for 18 hours, followed by cutting out and lyophilizing the stained gel bands.

The dried gel bands were soaked in a 0.6 ml mixture solution consisting of 2 µg/ml **"TPCK TRYPSIN",** a trypsin preparation commercialized by Sigma Chemical Company, St. Louis, Missouri, USA, 0.5 mM calcium chloride and 0.02 v/v aqueous tween 20 solution, and incubated at 37°C for 18 hours to trypsinize the L-asparaginase. The trypsinized product was centrifuged and separated to obtain a supernatant and a precipitate which was then soaked in one ml of one v/v % aqueous trifluoroacetic acid solution containing 0.001 v/v % tween 20, stirred at ambient temperature for 4 hours, and centrifuged to obtain a supernatant. The resulting precipitate was successively treated similarly as above with a 70 v/v % aqueous trifluoroacetic acid solution containing 0.001 v/v % tween 20, and an acetonitrile solution containing 0.001 v/v % tween 20 and 50 v/v % trifluoroacetic acid to obtain a supernatant which was then mixed with the above supernatant. The mixture solution was concentrated up to give 250 µl which was then centrifugally filtered.

The aqueous solution containing the peptide fragments thus obtained was fed to a column of **"HPLC ODS-120T" ,** a gel for high-performance liquid column chromatography commercialized by Tosoh Corporation, Tokyo, Japan. The column was washed with 0.1 v/v % aqueous trifluoroacetic acid solution and fed with a linear gradient buffer of aqueous acetonitrile increasing from 0 v/v % to 70 v/v % in 0.1 v/v % aqueous trifluoroacetic acid solution at a flow rate of 0.5 ml/min. Fractions eluted at about 66 min, about 69 min and about 72 min after starting the feeding (hereinafter designated as **"peptide fragment A", "peptide fragment B"** and **"peptide fragment C"**, respectively) were collected. The elution pattern, i.e a peptide map of the guinea pig's L-asparaginase, was in FIG.1.

By using **"MODEL 473A"**, a protein sequencer commercialized by Perkin-Elmer Corp., Norwalk, USA, these peptide fragments A, B and C were studied in a conventional manner, revealing that they have the amino acid sequences of SEQ ID NOs:7-9.

### Example 3

### Total amino acid sequence of guinea pig L-asparaginase and base sequence coding for it

### Example 3-1

### Preparation of total RNAs

Three g of wet cells of a guinea pig liver, prepared in a conventional manner, was weighed, suspended in 20 ml of a 6 M aqueous guanidine isothiocyanate solution containing 10 mM sodium citrate (pH 7.0) and 0.5 w/v % SDS, and disrupted by a homogenizer. Into a 35-ml centrifuge tube was injected 25 ml of a mixture solution of 5.7 M cesium chloride and 0.1 M EDTA (pH 7.5), and the disrupted cells were overlaid on the solution, followed by centrifuging the tube at 25,000 rpm and at 20°C for 20 hours. A fraction containing RNAs was collected from the tube, transferred to another 15-ml centrifuge tube, mixed with an equal amount of chloroform/butanol (=4:1 by volume), stirred for 5 min, and centrifuged at 10,000g x 10 min and at 4°C. The resulting water layer was collected, mixed with 2.5-fold volumes of ethanol, and allowed to stand at -20°C for 2 hours to precipitate the total RNAs. The precipitated RNAs were washed with 75 v/v % ethanol and dried to obtain the total RNAs in a yield of about 4 mg.

### Example 3-2

### Preparation of DNA fragment partially coding for guinea pig L-asparaginase

To one µg of the total RNAs prepared in Example 3-1 were added 4 µl of 25 mM magnesium chloride, 2 µl of 10xPCR buffer consisting of 100 M Tris-HCl buffer (pH 8.3) and 500 mM potassium chloride, 8 µl of one mM dNTP mix, one µl of one unit/µl of an RNase inhibitor, one µl of 2.5 units/µl of a reverse transcriptase, one µl of 2.5 µM of a random hexamer, and a volume of sterile distilled water sufficient to bring the total volume to 20 µl. The mixture solution was placed into a 0.5-ml test tube and in a conventional manner successively incubated at 25°C for 10 min, at 42°C for 30 min, at 99°C for 5 min, and at 5°C for 5 min to conduct an enzymatic reverse transcriptase reaction. Thus, an aqueous solution containing a first strand cDNA was obtained.

Twenty µl of the aqueous solution was mixed with 4 µl of 25 mM magnesium chloride, 8 µl of 10xPCR buffer consisting of 100 mM Tris-HCl buffer (pH 8.3) and 500 mM potassium chloride, 0.5 µl of 2.5 units/ml of an amplitaque DNA polymerase, one pmol of primer 1 as a sense primer, one pmol of a primer 2 as an antisense primer, and a volume of sterile distilled water sufficient to bring the total volume to 100 µl. The mixture solution was in a conventional manner successively allowed to react at 94°C for one min, at 42°C for 2 min, and at 72°C for 3 min, and these sequential reaction steps were repeated 40 times to amplify a DNA fragment, which partially encodes the guinea pig L-asparaginase, by using the first strand cDNA as a template. The primers 1 and 2 are oligonucleotides, which were chemically synthesized based on the amino acid sequences of Gly-Met-Gln-Ser-Lys and Tyr-Pro-Gly-Ile-Pro-Ala in SEQ ID NOs:8 and 7, and have base sequences represented by 5'-GGNATGCARWSNAAR-3' and 5'-GCNGGDATNCCNGGRTA-3', respectively.

The PCR reaction product thus obtained was sampled and electrophoresed in a 2 w/v % agarose gel to effect fractionation, followed by press-blotting the gel with 0.4 N sodium hydroxide to transfer and fix DNAs onto a nylon film. The film was successively washed with 2xSSC, air-dried, soaked in a mixture solution for prehybridization containing 5xSSPE, 5xDenhardt's solution, 0.5 w/v % SDS and 100 µg/ml of a denatured salmon sperm DNA, and incubated in the solution at 65°C for 3 hours. An oligonucleotide as a probe 1, having a base sequence represented by 5'-TTYATGYTNGARAAYYT-3', was chemically synthesized based on the amino acid sequence of Phe-Met-Leu-Glu-Asn-Leu in SEQ ID NO:9, and labelled with [γ-³²P]ATP and T4 polynucleotide kinase. The above nylon film was successively soaked in a mixture solution containing one pmol of the probe 1, 5xSSPE, 5xDenhardt's solution, 0.5 w/v % SDS, and 100 µg/ml of a denatured salmon sperm DNA, incubated at 42°C for 24 hours to effect hybridization, washed with 6xSSC at ambient temperature, and autoradiographed in a conventional manner, revealing that the formed PCR product contained the objective DNA fragment.

The remaining PCR product was admixed with 50 ng **"pT7 BLUE T",** a plasmid vector commercialized by Novachem Ltd., Tel Aviv, Israel, and an adequate amount of T4 DNA ligase, admixed with 100 mM ATP up to give a final concentration of one mM, and incubated at 16°C for 18 hours to insert the above DNA fragment into the plasmid vector. The recombinant DNA thus obtained was introduced into "NoVa Blue strain", a microorganism of the species *Escherichia coli* commercialized by Novachem Ltd., Tel Aviv, Israel, to obtain a transformant which was then inoculated into a plate medium containing 10 g/ℓ bacto-tryptone, 5 g/ℓ bacto-yeast extract, 2.5 g/ℓ sodium chloride, 15 g/ℓ bacto-agar, 100 mg/ℓ ampicillin, 40 mg/ℓ X-Gal and 23.8 mg/ℓ isopropyl-β-D-thiogalactopyranoside (hereinafter abbreviated as "IPTG"), and incubated at 37°C for 24 hours to form colonies. A nylon film was in a conventional manner placed on the surface of the plate medium, allowed to stand for about 30 sec to transfer the colonies onto the film, then the film was detached from the plate medium and soaked in a mixture solution containing 0.5 M sodium hydroxide and 1.5 M sodium chloride for 7 min to lyse the cells. Thereafter, the nylon film was successively soaked in 0.5 M Tris-HCl buffer (pH 7.2) containing 1.5 M sodium chloride for 3 min, washed with 2xSSC, soaked in 0.4 M sodium hydroxide for 20 min, washed with 5xSSC, air-dried, soaked in a mixture solution for prehybridization containing 5xSSPE, 5xDenhardt's solution, 0.5 w/v SDS and 100 µg/ml of a denatured salmon sperm DNA, and incubated at 42°C for 24 hours. The probe 1 was hybridized with the nylon film in a conventional manner, and the resultant was washed with 6xSSC and autoradiographed similarly as above, followed by collecting from the plate medium a transformant which strongly hybridized with the probe 1.

The transformant was inoculated into L-broth (pH 7.2) containing 100 µg/ml ampicillin and cultured at 37°C for 18 hours, followed by collecting cells from the culture and collecting a recombinant DNA by conventional alkali-SDS method. The dideoxy method revealed that the recombinant DNA contained a DNA fragment of a base sequence corresponding to the bases 61-746 in SEQ ID NO:5.

### Example 3-3

### Preparation of mRNA

0.05 ml of an aqueous solution containing 500 µg of the total RNAs in Example 3-1 was placed in a container, into which was poured 0.5 ml of 10 mM Tris-HCl buffer (pH 7.5) containing one mM EDTA and 0.1 w/v % SDS up to give a total volume of one ml. To the mixture solution was added one ml of **"OLIGOTEX-dT300 SUPER",** an oligo(dT)₃₀ latex commercialized by Nippon Roche K.K., Tokyo, Japan, and the resulting mixture was incubated at 65°C for 5 min to denature the contents and promptly cooled in an ice-chilled bath for 3 min. 0.2 ml of 5 M sodium chloride was added to the cooled solution, and the resulting solution was incubated at 37°C for 10 min and centrifuged at 10,000 rpm and at 25°C for 10 min, followed by removing a supernatant to obtain a pellet-like precipitate, suspending the precipitate in 0.5 ml sterile distilled water, incubating the suspension at 65°C for 5 min to extract the objective mRNA from the **"OLIGOTEX-dT300 SUPER".** The yield of the mRNA was about 5 µg.

### Example 3-4

### Preparation of cDNA library

By using **"cDNA SYNTHESIZING SYSTEM PLUS",** a cDNA cloning kit commercialized by Amersham Corp., Div., Amersham International, Arlington Heights, USA, a cDNA library was prepared from the mRNA in Example 3-3. Into a 1.5-ml reaction tube were successively poured a 4 µl solution for synthesizing a first strand, one µl sodium pyrophosphate, one µl human placenta ribonuclease inhibitor, 2 µl dideoxynucleotide triphosphate mixture, and one µl oligo dT primer, then mixed with 5 µl of the mRNA prepared in Example 3-3 and a volume of sterile distilled water sufficient to bring the total volume to 19 µl. To the resulting solution added one µl of a solution containing 20 units of a reverse transcriptase, followed by incubating at 42°C for 40 min to obtain a reaction mixture containing a first strand cDNA.

The reaction mixture was successively admixed with a 37.5 µl solution for synthesizing a second strand cDNA, 0.8 units of ribonuclease H from *Escherichia coli,* and 23 units of DNA polymerase I, increased its volume up to 100 µl by the addition of sterile distilled water, incubated at 12°C for 60 min and at 22°C for 60 min, admixed with 2 units T4 DNA polymerase, and further incubated at 37°C for 10 min to obtain a solution containing the objective second strand cDNA. To the solution thus obtained was added 4 µl of 0.25 M EDTA (pH 8.0) to suspend the reaction, followed by extracting with phenol/chloroform, precipitating the objective cDNA with ethanol, and collecting the cDNA.

Two µl L/K buffer, 250 pmol *Eco* RI adapter and 2.5 units of T4 DNA ligase were added to the above cDNA in this order, and the mixture solution was increased its volume up to 20 µl with sterile distilled water, and incubated at 15°C for 16 hours to ligate the *Eco* RI adaptor to both ends of the cDNA. The reaction mixture was mixed with 2 µl of 0.25 M EDTA (pH 8.0) to inactivate the remaining enzymes, and the intact *Eco* RI adaptor was in a conventional manner removed by molecular sieve chromatography. To the resultant were added 40 µl L/K buffer and 80 units of T4 polynucleotide kinase, and the mixture was increased its volume up to 400 µl with sterile distilled water, followed by incubating the mixture solution at 37°C for 30 min to phosphorize the 5'-terminus of *Eco* RI adaptor, and precipitating the reaction mixture with phenol/chloroform and ethanol to obtain a DNA. The DNA was mixed with 1.5 µl L/K buffer containing an adequate amount of λgt10 arm and with 2.5 units of T4 DNA ligase, increased its volume up to 15 µl with sterile distilled water, incubated at 15°C for 16 hours, and treated with conventional *in vitro* packaging method to obtain a phage containing a recombinant λDNA.

### Example 3-5

### Cloning of recombinant DNA

*Escherichia coli* NM514 strain, commercialized by Amersham Corp., Div., Amersham International, Arlington Heights, USA, was in a conventional manner infected with the phage in Example 3-4, inoculated into an agar plate (pH 7.0) containing 10 g/ℓ bacto-tryptone, 5 g/ℓ bacto-yeast extract, 10 g/ℓ sodium chloride, and 15 g/ℓ bacto-agar, and incubated at 37°C for 10 hours to form plaques. A nylon film was placed on the surface of the agar plate and allowed to stand for about 30 sec to transfer the plaques onto the film, then detached from the plate, and successively and repeatedly soaked in an aqueous solution containing 0.5 M sodium hydroxide and 1.5 M sodium chloride for 7 min and in 0.5 M Tris-HCl buffer (pH 7.0) containing 1.5 M sodium chloride for 3 min. The nylon film was successively rinsed with 5xSSC, air-dried, soaked in 0.4 N sodium hydroxide for 20 min, rinsed with 2xSSC, air-dried, soaked in a mixture solution containing 5xSSPE, 5xDenhardt's solution, 0.5 w/v % SDS and 100 µg/ml of a denatured salmon sperm DNA, and incubated at 65°C for 3 hours. The DNA fragment in Example 3-2 was labeled with ³²P using **"REDIPRIME DNA LABELLING SYSTEM"** Amersham Corp., Div., Amersham International, Arlington Heights, USA, to obtain a probe 2, and an adequate amount of which was hybridized at 65°C for 20 hours by incubating in a mixture solution containing 5xSSPE, 5xDenhardt's solution, 0.5 w/v % SDS and 100 µg/ml of a denatured salmon sperm DNA. The nylon film was successively soaked in and rinsed with 2xSSC at 65°C for 20 min and 0.2xSSC at 65°C for 20 min, and subjected to autoradiography, followed by selecting a phage DNA clone which strongly hybridized with the probe 2.

The clone was amplified in *Escherichia coli*, and a recombinant DNA was extracted from the microorganism. The recombinant DNA was cleaved by *Eco* RI, a restriction enzyme, and plasmid vector pUC19 (ATCC 37254) was cleaved with the same restriction enzyme. The formed DNA- and plasmid-fragments were in a conventional manner ligated with a DNA ligase to obtain a recombinant DNA which was then introduced into *Escherichia coli* JM109 strain (ATCC 53323) by conventional competent cell method to obtain a transformant.

### Example 3-6

### Determination of base sequence of DNA and amino acid sequence of protein

The transformant in Example 3-5 was inoculated into L-broth (pH 7.2) containing 50 µg/ml ampicillin and cultured at 37°C for 18 hours under shaking conditions. From the culture the proliferated transformants were collected and treated with conventional alkali-SDS method to obtain a recombinant DNA containing the DNA according to the present invention. The analysis of the recombinant DNA on an automatic sequencer using a fluorophotometer revealed that it contained the base sequence of SEQ ID NO:10, and the decoding indicated that it encodes the amino acid sequence of SEQ ID NO:10. The amino acid sequence of SEQ ID NO:10 has a partial amino acid sequence at positions 10-25 and positions 243-253 which is as shown in SEQ ID NO:8 and SEQ ID NO:7, respectively. The fact indicates that the DNA of SEQ ID NO:5 codes for a guinea pig L-asparaginase containing the amino acid sequence of SEQ ID NO:3.

In Example 4, a cDNA coding for human L-asparaginase is collected from a mRNA from human liver by using the DNA of SEQ ID NO:5 as a probe. The total amino acid sequence of human L-asparaginase was determined by analyzing and decoding the base sequence of the cDNA.

### Example 4

### Total amino acid sequence of human L-asparaginase and base sequence of DNA which codes for human L-asparaginase

### Example 4-1

### Preparation of cDNA library

By using "**"cDNA SYNTHESIZING SYSTEM PLUS",** a cDNA cloning kit commercialized by Amersham Corp., Div., Amersham International, Arlington Heights, USA, a cDNA library was prepared from a poly (A) modified human liver RNA commercialized by Clontech Laboratories, Inc., California, USA. Into a 1.5-ml reaction tube were poured 10 µl of a first strand synthesizing solution, 2.5 µl of one mM sodium pyrophosphate, 2.5 µl of one µg/µl of a human placenta ribonuclease inhibitor, 5 µl of one µg/µl deoxynucleotide triphosphate mixture, and 2.5 µl of one µg/µl oligo dT primer, then mixed with 5 µg of a poly (A) modified human liver RNA. The mixture was increased its volume up to 45 µl with sterile distilled water, then mixed with a 5 µl solution containing 100 units of a reverse transcriptase, and incubated at 42°C for 40 min to obtain a reaction product containing a first strand cDNA.

To the reaction product were added 93.5 µl of a cDNA synthesizing solution, 4 units of ribonuclease H from *Escherichia coli*, and 115 units of DNA polymerase I, and the mixture was increased its volume up to 250 µl with sterile distilled water, then successively incubated at 12°C for 60 min, at 22°C for 60 min, and at 70°C for 10 min, mixed with 10 units of T4 DNA polymerase, and further incubated at 37°C for 10 min, followed by suspending the reaction by the addition of 100 µl of 0.25 M EDTA (pH 8.0). The reaction product was in a conventional manner extracted with phenol/chloroform, and the extract was precipitated with ethanol to obtain a second strand cDNA.

The second strand cDNA thus obtained was mixed with 2 µl L/K buffer (pH 8.0), 250 pmol *Eco* RI adaptor, and 2.5 units T4 DNA ligase, and the mixture was increased its volume up to 20 µl with sterile distilled water and incubated at 15°C for 16 hours to ligate the *Eco* RI adaptor to both ends of the cDNA. The reaction was suspended by the addition of 2 µl of 0.25 M EDTA (pH 8.0). Intact Eco RI adaptor was removed from the reaction mixture by molecular sieve chromatography, and the remaining reaction mixture was mixed with 40 µl of L/K buffer (pH 8.0) and 80 units of T4 polynucleotide kinase, increased its volume up to 400 µl with sterile distilled water, incubated at 37°C for 30 min to phosphorize the 5'-terminus of the Eco RI adaptor, and extracted with phenol/chloroform. The extract was precipitated with ethanol to collect cDNA which was then mixed with 1. 5 µl of L/K buffer (pH 8.0) containing an adequate amount of λgt 10 arm and 2.5 units of T4 DNA ligase, and the mixture was increased its volume up to 15 µl with sterile distilled water, incubated at 15°C for 16 hours, and applied with the *in vitro* packaging to obtain a phage containing a recombinant λDNA.

### Example 4-2

### Cloning of recombinant DNA

*Escherichia coli* NM514 strain was infected with the phage in Example 4-1, inoculated into an agar plate (pH 7.0) containing 10 g/ℓ bacto-tryptone, 5 g/ℓ bacto-yeast extract, 10 g/ℓ sodium chloride and 15g/ℓ bacto-agar, and incubated at 37°C for 10 hours to form plaques. A nylon film was placed on the surface of the agar plate, allowed to stand for about 30 sec to transfer the plaques onto the film, detached from the plate, and successively and repeatedly soaked in an aqueous solution containing 0.5 M sodium hydroxide and 1.5 M sodium chloride for 7 min, and 0.5 M Tris-HCl buffer (pH 7.0) containing 1.5 M sodium chloride for 3 min. The film was rinsed with 2xSSC, air-dried, soaked in a mixture solution containing 5xSSPE, 5xDenhardt's solution, 0.5 w/v % SDS and 100 µg/ml of a denatured salmon sperm, and incubated at 65°C for 3 hours.

To clone a recombinant DNA a probe 3 was prepared by labelling the DNA fragment of SEQ ID NO:5 with **"REDIPRIME DNA LABELLING SYSTEM":** Twenty-five ng of a DNA fragment, obtained by the method in Example 3-5, was.placed in a 1.5-ml reaction tube, mixed with sterile distilled water to obtain 45 µl solution which was then heated at 95°C for 3 min, and transferred to another reaction tube. Five µl of [α-³²P]dCTP solution was added to the tube, and the DNA fragment was labelled by incubating at 37°C for 30 min. The reaction mixture containing the labelled DNA fragment was treated with conventional molecular sieve chromatography to remove intact [α-³²P]dCTP. Thus the probe 3 was obtained.

The nylon film was incubated at 50°C for 20 hours in a mixture solution containing an adequate amount of the probe 3, 5xSSPE, 5xDenhardt's solution, 0.5 w/v % SDS, and 100 µg/ml of a denatured salmon sperm to hybridize the probe, then successively incubated in 6xSSC at ambient temperature for 20 min, incubated in 2xSSC at ambient temperature for 20 min, washed and autoradiographed, followed by selecting a phage clone which strongly hybridized with the probe 3. The clone was in a conventional manner amplified in *Escherichia coli*, then a recombinant DNA was extracted from the microorganism. The recombinant DNA was cleaved with *Eco* RI, a restriction enzyme, and in a conventional manner ligated by a DNA ligase with the DNA fragment and a plasmid fragment, obtained by cleaving plasmid vector pUC19 (ATCC 37254) with the restriction enzyme. The recombinant DNA was introduced into *Escherichia coli* JM109 strain (ATCC 53323) by conventional competent cell method to obtain a transformant. The hybridization conditions outlined above may be described as stringent conditions, particularly for the purposes of this invention.

### Example 4-3

### Determination of base sequence of DNA which codes for human L-asparaginase, and its total amino acid sequence

The transformant in Example 4-2 was inoculated into L-broth (pH 7.2) containing 50 µg/ml ampicillin and cultured at 37°C for 18 hours under shaking conditions. From the culture the proliferated transformants were collected, treated with conventional alkali-SDS method to obtain the present recombinant DNA according to the present invention. The analysis of the recombinant DNA on an automatic sequencer using a fluorophotometer revealed that it has the base sequence of SEQ ID NO:11. An amino acid sequence estimable from the base sequence is also in SEQ ID NO:11, and this indicates that the DNA of SEQ ID NO:6 codes for human L-asparaginase containing the amino acid sequence of SEQ ID NO:4. Comparison of the amino acid sequences of SEQ ID NOs:3 and 4 revealed that they have an about 70% homology.

As is described above, the present invention is based on the finding of a novel DNA coding for a mammalian L-asparaginase. The application of conventional DNA technology to the present DNA facilitates to produce a desired amount of mammalian L-asparaginases including human's which could not have been readily obtained.

The present invention with these outstanding effects would greatly contribute to this filed.

While there has been described what is at present considered to be the preferred embodiments of the invention, it will be understood the various modifications may be made therein, and it is intended to cover in the appended claims all such modifications as fall within the true spirit and scope of the invention.

## Claims

1. A DNA coding for mammalian L-asparaginase, **characterised in that** said DNA comprises a base sequence which encodes both amino acid sequences as shown in SEQ ID NO:1 and SEQ ID NO:2 and said DNA is hybridisable with either or both DNA comprising a base sequence as shown in SEQ ID NO:5 or DNA comprising a base sequence as shown in SEQ ID NO:6 at 50°C in a solution of 5 x SSPE, 5 x Denhardt's solution, 0.5 w/v % SDS, and 100 µg/ml of a denatured salmon sperm.

2. A DNA according to claim 1, **characterised in that** said DNA comprises a base sequence which encodes either an amino acid sequence as shown in SEQ ID NO:3 or a homologous amino acid sequence to the amino acid sequence shown in SEQ ID NO:3.

3. A DNA according to claim 1, **characterised in that** said DNA comprises a base sequence which encodes either an amino acid sequence as shown in SEQ ID NO: 4 or a homologous amino acid sequence to the amino acid sequence shown in SEQ ID NO: 4.

4. A DNA according to claim 2, **characterised in that** said DNA comprises a base sequence that is either as shown in SEQ ID NO:5 or a homologous base sequence to the base sequence shown in SEQ ID NO:5 or a complimentary base sequence to the base sequence shown in SEQ ID NO:5

5. A DNA according to claim 3, **characterised in that** said DNA comprises a base sequence that is either as shown in SEQ ID NO:6 or a homologous sequence to the base sequence shown in SEQ ID NO:6 or a complimentary base sequence to the base sequence shown in SEQ ID NO:6.

6. The DNA according to claim 2, which has the base sequence of SEQ ID NO: 10:

7. The DNA according to claim 3, which has the base sequence of SEQ ID NO:11:

8. The DNA according to claim 4, wherein one or more bases in SEQ ID NO:5 are replaced with other bases by means of the degeneracy of genetic code without changing the amino acid sequence of SEQ ID NO:3.

9. The DNA according to claim 5, wherein one or more bases in SEQ ID NO:6 are replaced with other bases by means of the degeneracy of genetic code without changing the amino acid sequence of SEQ ID NO:4.

10. The DNA according to claim 1, which codes for an L-asparaginase of guinea pig or human.

## Patentansprüche

1. DNA, codierend für L-Asparaginase aus Säugern, **dadurch gekennzeichnet, daß** die DNA eine Basensequenz umfaßt, die die beiden in SEQ ID NO:1 und SEQ ID NO:2 gezeigten Aminosäuresequenzen codiert, wobei die DNA mit einer oder beiden der DNA, die eine wie in SEQ ID NO:5 gezeigte Basensequenz umfaßt, bzw. der DNA, die eine wie in SEQ ID NO:6 gezeigte Basensequenz umfaßt, in einer Lösung aus 5 x SSPE, 5 x Denhardts-Lösung, 0,5% (w/v) SDS und 100 µg/ml eines denaturierten Lachsspermas bei 50°C hybridisierbar ist.

2. DNA nach Anspruch 1, **dadurch gekennzeichnet, daß** die DNA eine Basensequenz umfaßt, die entweder eine wie in SEQ ID NO:3 gezeigte Aminosäuresequenz oder eine zur in SEQ ID NO:3 gezeigten Aminosäuresequenz homologe Aminosäuresequenz codiert.

3. DNA nach Anspruch 1, **dadurch gekennzeichnet, daß** die DNA eine Basensequenz umfaßt, die entweder eine wie in SEQ ID NO:4 gezeigte Aminosäuresequenz oder eine zur in SEQ ID NO:4 gezeigten Aminosäuresequenz homologe Aminosäuresequenz codiert.

4. DNA nach Anspruch 2, **dadurch gekennzeichnet, daß** die DNA eine Basensequenz umfaßt, bei der es sich entweder um die wie in SEQ ID NO:5 gezeigte Basensequenz oder um eine zur in SEQ ID NO:5 gezeigten Basensequenz homologe Basensequenz oder um eine zur in SEQ ID NO:5 gezeigten Basensequenz komplementäre Basensequenz handelt.

5. DNA nach Anspruch 3, **dadurch gekennzeichnet, daß** die DNA eine Basensequenz umfaßt, bei der es sich entweder um die wie in SEQ ID NO:6 gezeigte Basensequenz oder um eine zur in SEQ ID NO:6 gezeigten Basensequenz homologe Sequenz oder um eine zur in SEQ ID NO:6 gezeigten Basensequenz komplementäre Basensequenz handelt.

6. DNA nach Anspruch 2, die die Basensequenz der SEQ ID NO:10 aufweist:

7. DNA nach Anspruch 3, die die Basensequenz der SEQ ID NO:11 aufweist:

8. DNA nach Anspruch 4, wobei eine oder mehrere Basen in SEQ ID NO:5 gegen andere Basen mittels des degenerierten genetischen Codes ausgetauscht werden, ohne daß dabei die Aminosäuresequenz der SEQ ID NO:3 verändert wird.

9. DNA nach Anspruch 5, wobei eine oder mehrere Basen in SEQ ID NO:6 gegen andere Basen mittels des degenerierten genetischen Codes ausgetauscht werden, ohne daß dabei die Aminosäuresequenz der SEQ ID NO:4 verändert wird.

10. DNA nach Anspruch 1, die für eine L-Asparaginase aus Meerschweinchen oder Mensch codiert.

## Revendications

1. ADN codant pour une L-asparaginase de mammifère, **caractérisé en ce que** ledit ADN comprend une séquence de bases qui code pour les deux séquences d'acides aminés telles que représentées par la SEQ. ID. N° 1 et la SEQ. ID. N° 2, et ledit ADN est hybridable avec un ADN comprenant une séquence de bases telle que représentée par la SEQ. ID. N° 5 et/ou avec un ADN comprenant une séquence de bases telle que représentée par la SEQ. ID. N° 6 à 50°C dans une solution de 5 x SSPE, 5 x solution de Denhardt, 0,5 % en poids/volume de SDS et 100 µg/ml de sperme de saumon dénaturé,

2. ADN selon la revendication 1, **caractérisé en ce que** ledit ADN comprend une séquence de bases qui code soit pour une séquence d'acides aminés telle que représentée par la SEQ. ID. N° 3, soit pour une séquence d'acides aminés homologue à la séquence d'acides aminés représentée par la SEQ. ID. N° 3,

3. ADN selon la revendication 1, **caractérisé en ce que** ledit ADN comprend une séquence de bases qui code soit pour une séquence d'acides aminés telle que représentée par la SEQ. ID. N° 4, soit pour une séquence d'acides aminés homologue à la séquence d'acides aminés représentée par la SEQ. ID. N° 4,

4. ADN selon la revendication 2, **caractérisé en ce que** ledit ADN comprend une séquence de bases qui est soit telle que représentée par la SEQ. ID. N° 5, soit une séquence de bases homologue à la séquence de bases représentée par la SEQ. ID. N° 5, soit une séquence de bases complémentaire de la séquence de bases représentée par la SEQ. ID. N° 5.

5. ADN selon la revendication 3, **caractérisé en ce que** ledit ADN comprend une séquence de bases qui est soit telle que représentée par la SEQ. ID. N° 6, soit une séquence de bases homologue à la séquence de bases représentée par la SEQ. ID. N° 6, soit une séquence de bases complémentaire de la séquence de bases représentée par la SEQ. ID. N° 6.

6. ADN selon la revendication 2, qui a la séquence de bases de la SEQ. ID. N° 10,

7. ADN selon la revendication 3, qui a la séquence de bases de la SEQ. ID. N° 11,

8. ADN selon la revendication 4, dans lequel une ou plusieurs bases de la SEQ. ID. N° 5 sont remplacées par d'autres bases au moyen de la dégénérescence du code génétique, sans modifier la séquence d'acides aminés de la SEQ. ID. N° 3.

9. ADN selon la revendication 5, dans lequel une ou plusieurs bases de la SEQ. ID. N° 6 sont remplacées par d'autres bases au moyen de la dégénérescence du code génétique, sans modifier la séquence d'acides aminés de la SEQ. ID. N° 4.

10. ADN selon la revendication 1, qui code pour une L-asparaginase de cobaye ou d'être humain.
